Europäisches Patentamt

European Patent Office

Office européen des brevets

(19)

(11) Numéro de publication: **0 400 157**
**A2**

# (12) DEMANDE DE BREVET EUROPEEN

(21) Numéro de dépôt: **88100834.6**

(51) Int. Cl.5: **A61N 1/365**

(22) Date de dépôt: **20.07.84**

Cette demande a été déposée le 21.01.1988 comme demande divisionnaire de la demande mentionnée sous le code INID 60.  ·

(30) Priorité: **02.08.83 FR 8312740**

(43) Date de publication de la demande:
**05.12.90 Bulletin 90/49**

(60) Numéro de publication de la demande initiale en application de l'article 76 CBE : **0 133 828**

(84) Etats contractants désignés:
**AT BE CH DE GB IT LI LU NL SE**

(71) Demandeur: BIOVALLEES Société Anonyme:

**F-43150 Monastier sur Gazelle(FR)**

(72) Inventeur: **Brehier, Jacques**
**42, rue P. Belon**
**F-72000 Le Mans(FR)**

(74) Mandataire: **Derambure, Christian**
**BUGNION ASSOCIES 55, rue Boissonade**
**F-75014 Paris(FR)**

(54) **Sonde de stimulateur cardiaque.**

(57) La sonde de l'invention est destinée à un stimulateur cardiaque.

Elle comprend à sa partie extrême libre une extrémité de stimulation (23).

De plus elle comprend des moyens de mesure de la température telle qu'une thermistance (25) placée à la surface extérieure de la gaine (24a) entourant la sonde.

FIG.2

EP 0 400 157 A2

## SONDE DE STIMULATEUR CARDIAQUE

La présente invention concerne les sondes des stimulateurs cardiaques implantables.

Les stimulateurs cardiaques implantables sont réglés ou programmés pour stimuler le coeur d'un patient selon une certaine fréquence fixée. Il existe certains stimulateurs cardiaques qui peuvent être reprogrammés par voie externe pour différentes fréquences de stimulation. cependant, les stimulateurs cardiaques connus ne sont pas conçus pour être adaptés au degré d'activité physique réelle du sujet porteur chez qui le stimulateur est implanté. Cela signifie que si le patient exerce une activité physique importante, la fréquence de programmation préfixée peut se révéler insuffisante avec les conséquences de fatigue du patient qui en résultent. Au contraire, lorsque par exemple, le patient est au repos, ou endormi, la fréquence de stimulation préprogrammée peut être excessivement élevée, de façon superflue, voire dangereuse. Ainsi, la fréquence de stimulation choisie choisie est un compromis qui n'autorise, à partir de la valeur choisie qu'une faible variation du degré d'activité physique du patient.

L'invention a pour objectif concourir à remédier à ces inconvénients.

Certains stimulateurs utilisent la mesure de la température du patient, plus particulièrement celle du sang dans le coeur. Pour permettre cette mesure, une sonde a été décrite, par exemple dans le document DE-2.609.365, qui comporte un organe de mesure de la température.

L'invention concerne une sonde destinée à être associée à un stimulateur cardiaque qui comporte des moyens de mesure de la température, par exemple une thermistance plane à la surface extérieure de la gaine entourant la sonde.

L'invention sera bien comprise grâce à la description qui suivra, en référence aus dessins annexés dans lesquels :

-la figure 1 est un bloc diagramme de la partie électronique d'un stimulateur cardiaque implantable, selon l'invention,

-la figure 2 est une vue en coupe longitudinale illustrant la partie extrême de stimulation d'une sonde associée à un stimulateur cardiaque.

-la figure 3 est un diagramme illustrant la relation entre la température mesurée et la fréquence de stimulation dans un stimulateur cardiaque selon l'invention.

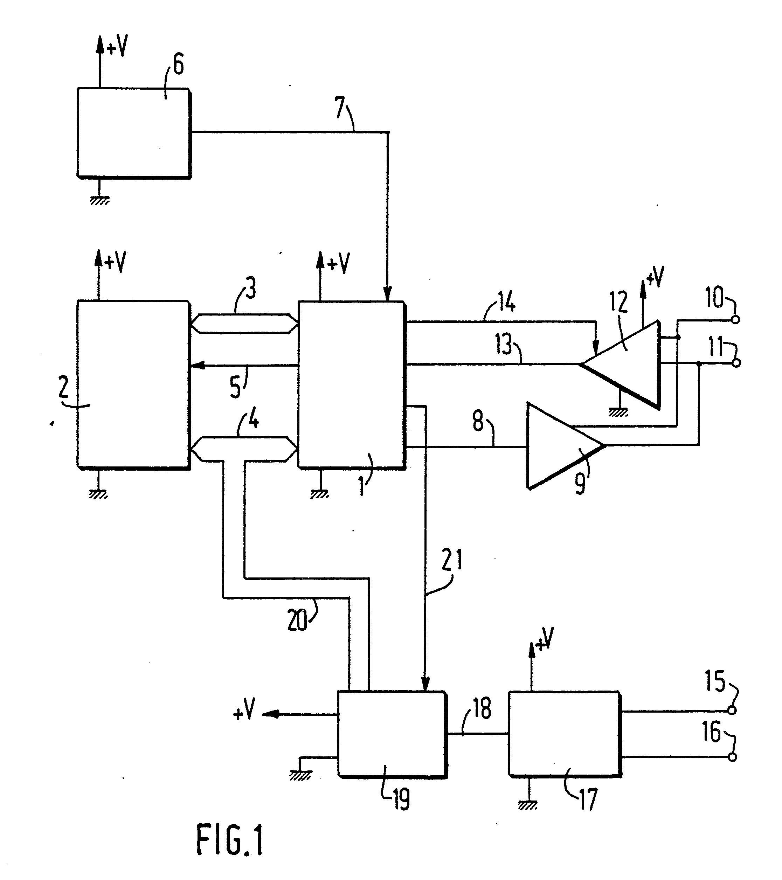

Un stimulateur cardiaque, selon l'invention comprend, outre les éléments conventionnnels connus, le circuit électronique représenté sur la figure 1 comportant un microprocesseur 1 connecté à une mémoire de lecture 2 au moyen d'un bus d'adresses 3, un bus de données 4 et une ligne de commande 5. Une horloge 6 génératrice d'impulsion et reliée par une ligne 7 à une entrée du microprocesseur 1. Le microprocesseur 1 a une ligne de sortie 8 reliée à l'entrée d'un amplificateur de stimulation 9 dont les sorties sont reliées aux bornes 10 et 11 auxquelles est branchée und sonde de stimulation (sa partie électrique de stimulation).

Les bornes 10 et 11 sont également reliées aux entrées d'un d'un amplifacteur de détection 12 dont la sortie est reliée par une ligne 13 au microprocesseur 1. Le microprocesseur 1 a également une sortie reliée par une ligne 14 à une entrée de commande de l'amplificateur de détection 12.

Deux entrées 15 et 16 sont prévues pour être reliées à une thermistance décrite ultérieurement. Les entrées 15 et 16 sont également reliées aux entrées d'un convertisseur analogique-digital 17. La sortie du convertisseur 17 est reliée par une ligne 18, à l'entrée d'un compteur 19. Les sorties du compteur 19 sont reliées par un bus 20 au bus de données 4. Enfin, le compteur 19 est commandé par le microprocesseur 1 par une ligne 21.

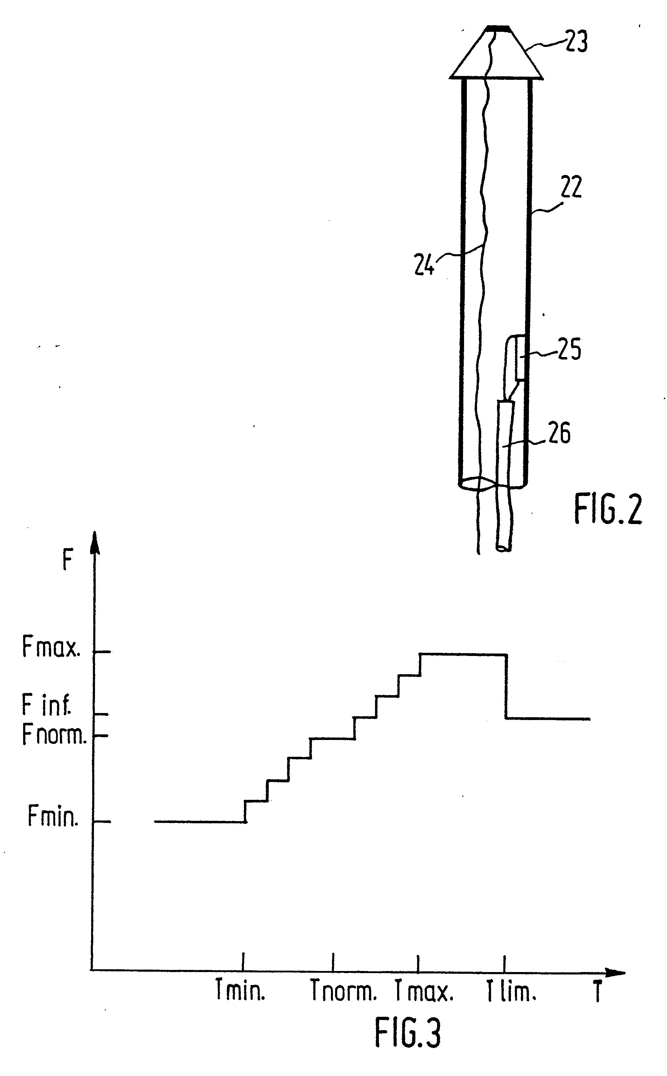

Sur la figure 2 est représentée la partie extrême libre de stimulation d'une sonde 22 comportant une extrémité de stimulation 23. Celle-ci est reliée à l'une des bornes 10, 11, par l'intermédiaire d'un fil conducteur spiralé 24, l'ensemble étant placé dans une gaine 24a. L'autre borne 10. 11 peut être reliée à une autre électrode, par exemple le boîtier de stimulateur cardiaque formant masse. A la surface externe de la gaine 24a est placée une thermistance 25 faisant partie intégrante de la sonde 22. Cette thermistance 25 est placée, de telle manière qu'elle permet une mesure effective du corps qui l'environne, par exemple le sang du coeur du patient. A cette thermistance 25 sont branchés des conducteurs 26 également noyés dans la gaine 24a, connectés aux bornes 15 et 16 (figure 1).

Préférentiellement, la thermistance 25 est écartée de l'extrémité de stimulation 23, par exemple d'une distance de l'ordre de 10 cm. ce qui permet une mesure correcte de la température, la thermistance étant placée dans l'oreillette droite à proximité immédiate de l'abouchement de la veine cave inférieure.

Le résistance de la thermistance 25 varie en fonction de la température du média qui l'environne. Cela provoque une variation du courant d'entrée du convertisseur 17 qui cause une variation correspondante de la fréquence du signal de sortie du convertisseur 17. Le microprocesseur 1 échantillonne la température en activant le compteur 19, via la ligne de commande 21 pendant une période prédéterminée. La lecture en sortie du compteur 19 est transférée au microprocesseur 1 via le bus 20 et le bus 4. Le microprocesseur 1 ajuste la fréquence de sortie pour l'amplificateur de stimulation 9 via la ligne 8, selon la valeur lue au compteur 19 et les fréquences de stimulation préprogrammées se trouvant dans le mémoire 2.

On se réfère maintenant à la figure 3 qui est un diagramme donnant en abscisses la température du sang T
- correspondant au degré d'activité physique du patient
- mesurée par la thermistance 25,
et en ordonnées, la fréquence de stimulation F. A une température Thorm qui est une température considérée normale pour le patient, correspond une fréquence de stimulation Fnorm. Si la temperature du patient augmente à partir de Tnorm, du fait de l'activité physique notamment, la fréquence de stimulation va également augmenter et ce jusqu'à une fréquence maximale Fmax correspondant à une température Tmax. De la même manière, si la température du sang diminue, à partir de la température Tnorm, la fréquence de stimulation diminue et ce jusqu'à une fréquence de stimulation minimale Fmin correspondant à une température Tmin. Si la température continue à diminuer à partir de Tmin, la fréquence restera inchangée à la valeur Fmin. D'autre part, si la température augmente à partir de la valeur Tmax, la fréquence restera inchangée à la valeur Fmax jusqu'à ce que la température atteigne une limite Tlim. A partir et au dessus de Tlim, la fréquence de stimulation est ramenée à une valeur inférieure à Fmax, soit Finf qui est, par exemple, de l'ordre de 20 % inférieure à la fréquence Fmax. Tlim peut être par exemple choisie entre 38°C et 39°C, préférentiellement égale ou voisine de 38,5°C (Tnorm étant égal ou de l'ordre de 37°C). La fréquence Finf peut être, par exemple inférieure de 15 à 25 % à la fréquence Fmax.

Il est clair que les températures Tlim, Tnorm, Tmax ainsi que les fréquences Flim, Fnorm, Fmax et Finf peuvent être préprogrammées ou réglées également par voie externe ou interne.

On peut également concevoir que la fonction liant la fréquence à la température puisse être programmée. Dans la variante illustrée sur la figure, cette fonction est illustrée, dans sa partie centrale, par un escalier, c'est-à-dire que la fréquence reste constante dans la plage de température et fait un saut lorsque la température atteint la borne de la plage. Mais, on peut concevoir également un réglage continu de la température.

Pour éviter la prise en compte de brusques variations de température, il peut être prévu des rayons aptes à effectuer une moyenne de température ou encore à lisser la courbe de variation de température.

## Revendications

1. Sonde de stimulation destinée à un stimulateur cardiaque selon l'une quelconque des revendications 6 à 9 comprenant à sa partie extrême libre une extrêmité de stimulation (23), du type comportant des moyens de mesure de température telle qu'une thermistance (25), caractérisée par le fait que la thermistance (25) est placée à la surface extérieure de la gaine (24a) entourant la sonde.

2. Sonde selon l'une quelconque des revendications 10 et 11, caractérisé par le fait que la thermistance (25) est écartée de l'extrémité de stimulation (23) d'une certaine distance par exemple de l'ordre d'approximativement 10cm permettant à la thermistance d'être placée dans l'oreillette droite.

3. Sonde selon l'une quelconque des revendications 10 à 12, caractérisée par le fait que la thermistance (25) est associée à des conducteurs électriques (26) destinés à être branchés aus bornes (15 et 16) du circuit électrique du stimulateur cardiaque.

FIG.1

FIG.2

FIG.3